# EUROPEAN PATENT APPLICATION

(11) **EP 1 645 628 A1**
(43) Date of publication of application: **12.04.2006**
(21) Application number: 05256214.7
(22) Date of filing: 05.10.2005
(51) Int. Cl.: C12N 15/10

(54) **Nanopore separation devices for biopolymers**

(30) Priority: 05.10.2004 US 959442
(71) Applicant: Agilent Technologies, Inc., Palo Alto, CA 94306 (US)
(72) Inventor: Li, Gangqiang, Palo Alto CA 94306 (US); Pittaro, Richard J., San Carlos CA 94070 (US)
(74) Representative: Tollett, Ian

(57) **Abstract**

The invention relates to nanopore-based separation devices and systems, and methods and kits for using these. The devices may be used to separate and evaluate biopolymers such as nucleic acids and proteins.

## Description

This application relates to nanopore separation devices and methods of using such devices.

The ability to separate, characterize, compare and isolate biopolymers has important applications in functional genomics and proteomics and in molecular diagnostic techniques. One method for separating biomolecules is electrophoresis. Electrophoresis typically relies on the application of an electric current to a medium containing a mixture of biological molecules. Molecules travel through the medium at a rate dependent on electrical charge and size and can be separated based on differences in these parameters. Agarose and acrylamide are commonly used for electrophoresis of nucleic acids and proteins.

Capillary electrophoresis, in which separation of molecules occurs in a narrow bore capillary tube subjected to a high voltage (e.g., 5-15 kV), employs the use of an electrolyte solution that conducts current through the inside of the capillary. A small volume of sample is injected at one end of the capillary. A detector is positioned at the opposite end of the capillary to detect the presence of the various sample components as they travel through the capillary and past the detector. The sample may be labeled with a fluorescent marker so that when the sample components pass through a beam of light at the detector, the fluorescent marker fluoresces, and the fluorescence is detected as an electric signal. In traditional capillary electrophoresis systems, analysis or detection of the separated components is performed while the sample is still located within the capillary and may be accomplished using photometric techniques such as absorbance and fluorescence. Absorbance and fluorescence is where excitation light is directed toward the capillary tube, and light emitted from the sample (e.g., fluorescence) is measured by a detector, thereby providing information about the separated components. A drawback of such a system is that the tubular shape of the fused silica capillaries causes significant scattering of light interfering with detection of samples.

Microfluidic chips may be used to perform electrophoresis, using channels formed in a substrate rather than capillary tubes. Microfluidic chips are amenable to high throughput approaches; however, on-chip detection may still be problematic where excitation light is scattered by materials used in forming the chip.

According to a first aspect of the present invention, there is provided a method for separating biopolymers as claimed in claim 1.

In an embodiment the biopolymer comprises a nucleic acid molecule. The nucleic acid molecule may be single-stranded or may be at least partially double-stranded.

In an embodiment, the biopolymer comprises a polypeptide.

In an embodiment, the plurality of biopolymers comprises at lest two of: polypeptides, DNA, RNA, and carbohydrates and the method comprises translocating biopolymers selected from the group consisting of polypeptides, DNA, RNA, and carbohydrates into the second reservoir.

In an embodiment, the method further comprises removing the collected biopolymer from the first or second reservoir after the translocation.

The method may comprise removing biopolymers from the first reservoir to a first container and removing biopolymers from the second reservoir to a second container after the translocation.

The method may further comprise the step of determining the sequence of the removed biopolymers.

In an embodiment the electric field is established by applying a voltage bias across the nanopore, the voltage bias is controlled by a processor and the processor accesses a memory and data relating to a predetermined time stored in the memory.

The processor may be in communication with a user device and the user may input data relating to the predetermined time into a display of the user device.

The method may be used to separate nucleic acid fragments generated by contacting nucleic acid samples with a restriction enzyme.

In an embodiment, the biopolymer is bound to another molecule, the method comprises translocating bound polymer and the method comprises collecting bound molecules, separating the molecules which are bound and determining the sequence of one or both of the bound molecules.

The method may further comprise subjecting collected biopolymer to mass spectrometry.

According to a second aspect of the present invention there is provided a device as claimed in claim 18.

The processor may provide instructions to the voltage source to reverse polarity of an applied voltage after a period of time sufficient to translocate a biopolymer of a selected size across the nanopore.

In an embodiment, the device comprises a detector for measuring current over time, wherein the processor provides instructions to the voltage source to stop applying voltage or to reverse polarity of an applied voltage after a predetermined period of time after current decreases below a threshold level.

The predetermined time is preferably a time sufficient to translocate a biopolymer of a selected size.

According to a third aspect of the present invention, there is provided a computer program product as claimed in claim 29.

According to a fourth aspect of the present invention, there is provided a kit as claimed in claim 30.

According to a fifth aspect of the present invention, there is provided a system as claimed in claim 32.

The invention relates generally to the field of biopolymers and more particularly to devices, systems, methods, and kits for separation of biopolymers (e.g., such as nucleic acids and proteins) using nanopore structures. Nanopore-based separation technique according to the invention provide the opportunity to evaluate biopolymers at high-speed and with high throughput, while using minimal amounts of sample.

In one embodiment, the invention provides a method for separating biopolymers (e.g., such as nucleic acids or proteins). The method comprises providing a plurality of biopolymers in a first reservoir connected to a second reservoir by a nanopore, establishing an electrical field across the nanopore sufficient to translocate a biopolymer across the nanopore, thereby separating the biopolymer from other biopolymers, and collecting the translocated biopolymer in the second reservoir or biopolymers in the first reservoir. In one aspect, an electric field is applied for a sufficient amount of time to translocate a biopolymer of a selected size. In another aspect, polarity is reversed after the amount of time sufficient to translocate the biopolymer of a selected time. This process may be repeated one or multiple times until a substantially pure population of molecules comprising the selected size is present in the second reservoir.

In one embodiment, the first and second reservoirs are connected by a plurality of nanopores. In another embodiment, the first reservoir comprises a plurality of subreservoirs, each subreservoir connected to the second reservoir by a nanopore. In one aspect, an electric field is applied independently to at least two of the nanopores. In another aspect, biopolymers of different characteristics (e.g., such as different sizes) are translocated through at least two of the nanopores. The method may further comprise the step of removing collected biopolymer from the first and/or second reservoir after translocation. In one aspect, removed biopolymers are further processed (e.g., by concentrating, sequencing, mass spectrometry or other processing steps). The method may be used to separate biopolymers that differ by as little as one monomer (e.g., by one base pair in the case of a nucleic acid).

In one aspect, the voltage bias is controlled by a processor that receives input relating to an amount of time that has passed after a current across the nanopore begins to decrease below a threshold amount. The processor provides instructions to a voltage source applying the voltage after a predetermined time sufficient to translocate the biopolymer of the selected size has passed, to stop application of the voltage or to reverse polarity of the applied voltage. In one aspect, the processor has access to a memory and data relating to the predetermined time is stored in the memory. In another aspect, the processor is in communication with a user device and user inputs data relating to the predetermined time into a display or interface of a user device that communicates with the processor.

In certain aspects, the second reservoir is connected to a third reservoir by a nanopore and the method comprises establishing an electrical field across the nanopore connecting the second and third reservoir sufficient to translocate a biopolymer from the second reservoir across the nanopore into the third reservoir.

In one embodiment, the invention also provides a device comprising a first reservoir connectable to a second reservoir by a nanopore; a voltage source for generating an electric field across the nanopore; and a processor in communication with the voltage source; wherein the processor provides instructions to the voltage source to regulate the time period during which voltage is applied. In one aspect, the first reservoir is connectable to the second reservoir by a channel comprising the nanopore. In another aspect, the processor provides instructions to the voltage source to reverse polarity of an applied voltage after a period of time sufficient to translocate a biopolymer of a selected size across the nanopore. In a further aspect, the device comprises a detector for measuring current over time and the processor provides instructions to the voltage source to stop applying voltage or to reverse polarity of an applied voltage after a predetermined period of time after current decreases below a threshold level (e.g., a period of time sufficient to translocate a biopolymer of a selected size).

In one aspect, the first reservoir is connectable to the second reservoir by a plurality of nanopores. In another aspect, either or both first and second reservoirs comprise an outlet port for removing biopolymer. In a further aspect, the first and/or second reservoirs comprise channels. In still another aspect, the device comprises a connecting channel in fluid communication with the second reservoir.

In one aspect, the first reservoir comprises a plurality of subreservoirs, each subreservoir connectable to the second reservoir by a nanopore. A voltage may be independently applied across nanopores of at least two of the subreservoirs. In another aspect, the device comprises a third reservoir connectable to the second reservoir by a nanopore and a voltage may be applied independently across nanopores connecting first and second reservoirs and second and third reservoirs.

In one embodiment, the invention additionally provides a computer program product comprising a computer readable medium carrying program code for executing one or more functions of a processor in the device.

In another embodiment, the invention also provides a system comprising any of the aforementioned devices and a memory comprising a relational database that comprises data relating current impedance to size of biopolymers, wherein the processor has access to the memory.

In a further embodiment, the invention further provides a kit comprising any of the aforementioned devices and a biopolymer. In one aspect, the biopolymer comprises a known size. In another aspect, the kit comprises a plurality of biopolymers of known sizes. Additional reagents and compositions may be provided to perform any of the methods described above.

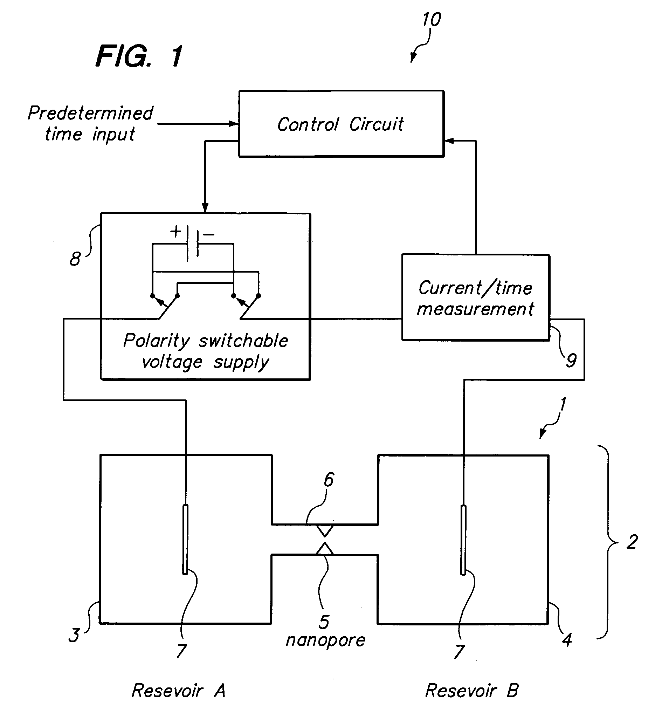

The objects and features of the invention can be better understood with reference to the following detailed description and accompanying drawings. The Figures shown herein are not necessarily drawn to scale, with some components and features being exaggerated for clarity. Note that FIGS. 1-4 show schematic representations of the present invention with open or exposed reservoirs and channels. Devices are not required to conform to these literal depictions. For instance, the reservoirs and channels may also be completely enclosed or within a substrate or other similar type material.

Embodiments of the invention are described below, by way of example only, with reference to the accompanying drawings, in which:
Figure 1 is a schematic diagram illustrating a system for biopolymer separation according to an aspect of the invention;
Figure 2 is a schematic diagram illustrating a system for parallel separation of biopolymers according to an aspect of the invention;
Figure 3 is a schematic diagram illustrating a system for sorted separation including a nanopore channel array according to an aspect of the invention;
Figure 4 is a schematic diagram illustrating a system for tandem separation of biopolymers according to an aspect of the invention; and
Figure 5 is a schematic diagram illustrating a system for sorted separation including a plurality of nanopore channels according to an aspect of the invention.

Before describing the present invention in detail, it is to be understood that this invention is not limited to specific compositions, method steps, or equipment, as such may vary. It is also to be understood that the terminology used herein is for the purpose of describing particular embodiments only, and is not intended to be limiting. Methods recited herein may be carried out in any order of the recited events that is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

Unless defined otherwise below, all technical and scientific terms used herein have the same meaning as commonly understood by one of ordinary skill in the art to which this invention belongs. Still, certain elements are defined herein for the sake of clarity.

All publications mentioned herein are incorporated herein by reference to disclose and describe the methods and/or materials in connection with which the publications are cited.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.

It must be noted that, as used in this specification and the appended claims, the singular forms "a", "an" and "the" include plural referents unless the context clearly dictates otherwise. Thus, for example, reference to "a biopolymer" includes more than one biopolymer, and reference to "a voltage source " includes a plurality of voltage sources and the like.

The following definitions are provided for specific terms that are used in the following written description.

A "biopolymer" is a polymer of one or more types of repeating units. Biopolymers are typically found in biological systems and particularly include polysaccharides (such as carbohydrates), peptides (which term is used to include polypeptides and proteins, such as antibodies or antigen-binding proteins), glycans, proteoglycans, lipids, sphingolipids, and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in hydrogen bonding interactions, such as Watson-Crick type, Wobble type and the like. In some cases the backbone of the biopolymer may be branched. Biopolymers may be heterogeneous in backbone composition thereby containing any possible combination of polymer units linked together such as peptide- nucleic acids (which have amino acids linked to nucleic acids and have enhanced stability). As used herein with respect to linked units of a biopolymer, "linked" or "linkage" means two entities are bound to one another by any physicochemical means. Such linkages are well known to those of ordinary skill in the art and include, but are not limited to, amide, ester and thioester linkages. Linkages include synthetic or modified linkages.

Polynucleotides include single or multiple stranded configurations, where one or more of the strands may or may not be completely aligned with another. A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides. Biopolymers include DNA (including cDNA), RNA, oligonucleotides, PNA, LNA and other polynucleotides as described in U.S. Patent No. 5,948,902 and references cited therein, regardless of the source.

Various techniques can be employed for preparing a polynucleotide. Such polynucleotides can be obtained by biological synthesis or by chemical synthesis. For short sequences (up to about 100 nucleotides), chemical synthesis is economical, provides a convenient way of incorporating low molecular weight compounds and/or modified bases during specific synthesis steps, and is very flexible in the choice of length and region of target polynucleotide binding sequence. Polynucleotides can be synthesized by standard methods such as those used in commercial automated nucleic acid synthesizers. Chemical synthesis of DNA on a suitably modified glass or resin can result in DNA covalently attached to the surface, potentially advantageous in washing and sample handling. For longer sequences standard replication methods employed in molecular biology can be used such as the use of M13 for single stranded DNA as described by Messing, J. (*Methods Enzymol.,* 1983, 101:20-78); or the use of polymerase chain reaction as described in U.S. Patent Nos. 4,683, 195; 4,683,202 and 4,965,188.

An "oligonucleotide" generally refers to a nucleotide multimer of about 10 to 100 nucleotides in length, while a "polynucleotide" includes a nucleotide multimer having any number of nucleotides.

A "biomonomer" references a single unit, which can be linked with the same or other biomonomers to form a biopolymer (e.g., a single amino acid or nucleotide with two linking groups one or both of which may have removable protecting groups).

As used herein, a "nanopore" refers to an opening in a material (biological or non-biological or some combination thereof) comprising at least on nanometer dimension. In one aspect, the term "nanopore" refers to any pore or hole between at least a pair of electrodes in a solid material. The dimensions of a nanopore may vary but are generally small enough to constrain passage of biopolymers through the nanopore such that only a single molecule may pass at a time. Nanopores can range in size, e.g., from about 1 nm to around about 300 nm. In certain embodiments, a nanopore is defined by the walls of a nanochannel that comprises the dimensions of a nanopore along at least a portion of the channel. The portion of the channel on either side of such a nanopore may define the first and second reservoirs, respectively, of the device.

A "substrate" refers to any surface that may or may not be solid and which is capable of holding, embedding, attaching or which may comprise the whole or portions of an electrode. As used herein, a "device comprising a housing" encompasses a substrate comprising a channel.

The term "in" refers to being "within" and/or a portion that may also be exterior to. For instance, a biopolymer "in" a nanopore may mean that the whole biopolymer is within the opening of the nanopore or only a small portion of the biopolymer is located near the nanopore with a substantial portion protruding exterior to the nanopore.

The term "translocation" or to "translocate" refers to movement from one side to another, movement in a defined direction. Any action or biopolymer following along a vector that has velocity and direction.

The term "portion" or "portion of a biopolymer" refers to a part, subunit, monomeric unit, portion of a monomeric unit, atom, portion of an atom, cluster of atoms, charge or charged unit.

The term "voltage gradient" refers to having the ability to establish a potential between any two electrodes.

The term "adjacent" refers to anything that is near, next to or adjoining. For instance, a nanopore may be near an electrode, it may be next to the electrode, it may pass through an electrode or it may be adjoining the electrode. This would include spacing in linear, two-dimensional and three-dimensional space.

The terms "hybridizing", "annealing" and "binding", with respect to polynucleotides, are used interchangeably. "Binding efficiency" refers to the productivity of a binding reaction, measured as either the absolute or relative yield of binding product formed under a given set of conditions in a given amount of time. "Hybridization efficiency" is a particular sub-class of binding efficiency, and refers to binding efficiency in the case where the binding components are polynucleotides.

It will also be appreciated that throughout the present application, that words such as "upper", "lower" are used in a relative sense only.

A "set" may have one type of member or multiple different types.

"Fluid" is used herein to reference a liquid.

The term "microfluidic chip," as use herein, includes not only a plastic or silicon or glass chip but also any substrate, such as a slide, wafer, disc, etc. capable of having a number of small channels, grooves, trenches, troughs, or analogous structures, formed therein for passing biopolymers.

The term "channel" as used herein refers to a passage through a substrate and is used interchangeably with the terms "groove," "trough," or trench." The geometry of a channel may vary widely and includes tubular passages with circular, rectangular, square, D-shaped, trapezoidal or other polygonal cross- sections. A channel may comprise varying channel geometries (e.g., rectangular at one section and trapezoidal at another section). Channels may form curved or angular paths through the substrate, and they may cross or intersect with other channels, and in various embodiments they can be substantially parallel to one another. In certain embodiments channels are filled with a separation media, reagents (e.g., such as enzymes, polymerases, antibodies, nucleic acids, polypeptides, peptides, and the like), and/or buffers

As used herein, the term "separation media" refers to a media in which an electrophoretic separation of sample components takes place. Separation media typically comprise several components, at !east one of which is a charge-carrying component or electrolyte. The charge- carrying component is usually part of a buffer system for maintaining the separation media at a defined pH. Media for separating polynucleotides, proteins, or other biomolecules having different sizes but identical charge-frictional drag ratios in free solution, further include a sieving component. Such sieving component is typically composed of a cross-linked polymer gel, e.g., cross-linked polyacrylamide or agarose, or a polymer solution, e.g., a solution of polyacrylamide, hydroxyethyl cellulose, and the like.

"Communicating information" refers to transmitting the data representing that information as signals (e.g., electrical, optical, radio, magnetic, etc) over a suitable communication channel (e.g., a private or public network).

As used herein, a component of a system which is "in communication with" or "communicates with" another component of a system receives input from that component and/or provides an output to that component to implement a system function. A component which is "in communication with" or which "communicates with" another component may be, but is not necessarily, physically connected to the other component. For example, the component may communicate information to the other component and/or receive information from the other component.

"Forwarding" an item refers to any means of getting that item from one location to the next, whether by physically transporting that item or otherwise (where that is possible) and includes, at least in the case of data, physically transporting a medium carrying the data or communicating the data.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture. In certain instances a computer-based system may include one or more wireless devices.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination which will perform the functions required of it. For example, any processor herein may be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

The term "assessing" and "evaluating" are used interchangeably to refer to any form of measurement, and includes determining if an element is present or not. The terms "determining," "measuring," and "assessing," and "assaying" are used interchangeably and include both quantitative and qualitative determinations. Assessing may be relative or absolute. "Assessing the presence of" includes determining the amount of something present, as well as determining whether it is present or absent.

The term "using" has its conventional meaning, and, as such, means employing, e.g. putting into service, a method or composition to attain an end. For example, if a program is used to create a file, a program is executed to make a file, the file usually being the output of the program. In another example, if a computer file is used, it is usually accessed, read, and the information stored in the file employed to attain an end. Similarly if a unique identifier, e.g., a barcode is used, the unique identifier is usually read to identify, for example, an object or file associated with the unique identifier.

### Devices Comprising Nanopores

In one embodiment, the invention provides a device I for performing biopolymer separations. In one aspect, the device comprises a housing 2 defining a first reservoir 3 and a second reservoir 4. The first reservoir 3 is connectable to the second reservoir 4 via a nanopore 5. In one aspect, a nanopore 5 is dimensioned so that only one biopolymer molecule (e.g., polynucleotide, polypeptide, etc.) may pass through at a time.

A biopolymer to be separated may be a nucleic acid, protein (e.g., an antibody), peptide, carbohydrate, glycan, lipid, sphingolipid, proteoglycan, etc, as discussed above. The shape or size of the molecule is not important, but it must be capable of translocation through a nanopore according to the invention. Nucleic acid biopolymers include RNA, DNA, or RNA-DNA complexes. Nucleic acid biopolymers may be single-stranded, double-stranded or partially double-stranded. Additional materials may be added to a biopolymer, such as metals, which may be used to modify the electrical properties of the biopolymer. For example, metals or materials may be added, doped, or intercalated with a biopolymer to provide a net dipole, a charge and/or to allow for conductivity through the biopolymer. The material of the biopolymer may allow for electron tunneling between optional electrodes placed adjacent to the nanopore opening. In one aspect, a biopolymer comprises a molecule or material which provides a way to track the position of the biopolymer in the first and second reservoir and/or as it translocates through the nanopore. In another aspect, a biopolymer is labeled directly or indirectly with a label which may or may not alter electrical properties of the biopolymer. In a further aspect, a biopolymer is labeled with a tag that increases its diameter and/or length. The tag may selectively bind to biopolymers having certain sequence characteristics (e.g., a selected nucleotide or amino acid sequence).

Nanopores may be generated from biological molecules (e.g., such as from a lipid bilayer on a solid support or from a pore-forming molecule (e.g., alpha-hemolysin) embedded in a lipid membrane, an ion channel, and the like) or from a non-biological material. In another aspect, a nanopore is generated from a solid-state material. Solid-state materials include, but are not limited to, organic and inorganic materials, such as microelectronic materials, insulating materials, such as Si₃N₄, Al₂O₃, SiO, organic and inorganic polymers such as polyamide, plastics such as Teflon®, elastomers such as silicone rubber, and glasses. Nanopores may be generated using integrated circuit (IC) fabrication techniques, and other methods, including, but not limited to, photolithography, e-beam iithography, epitaxial growth, ion beam sputtering, etching, delivery of TEM electron beams, and plasma enhanced chemical vapor deposition (PECVD). See, e.g., as described in U.S. Patent No. 6,706,204. See, also, Li et al.("Ion beam sculpting at nanometer length scales", *Nature,* 412: 166-169, 2001). Pores generated by one method may be altered size after initial fabrication, such as by etching of PECVD.

First and second reservoirs may have any shape or dimensions. In one aspect, the first and/or second reservoirs comprise channels in a substrate. The channels may range in size. In one aspect, channels are less than about 10 mm in width, less than about 5 mm in width, less than about 1 mm, less than about 100 nm, or even less than about 10 nm.

The first reservoir may comprise a port or inlet for adding solution to the reservoir, e.g., such as a solution comprising a population of biomolecules to be separated. In a further aspect, the second reservoir comprises a port or outlet for removing solution from the reservoir, such as solution comprising separated biopolymers having desired characteristics. The first reservoir also may comprise one or more outlet ports and the second reservoir may comprise one or more inlet ports.

The housing material may comprise a variety of materials known in the art for designing substrates and nanopores. The material may or may not be a solid material. For instance, the material may comprise a mesh, wire, or other material from which a nanopore may be constructed. Such materials may comprise silicon, silica, solid-state material such as Si₃N₄, silicon-rich silicon nitride, silicon oxynitride, carbon-based materials, plastics, polymers, resins, glasses, metals, or other materials known in the art for etching or fabricating semiconductor or electrically conducting materials, and combinations thereof. In certain aspects the housing is at least partially light transmissive. However, in another aspect, the housing is light impermeable (less than 10% of light passes through). In one aspect, the housing comprises a material comprising one or more layers, one of which may be a conducting layer. In another aspect, the device additionally comprises non-conducting material layers.

The housing may comprise various shapes and sizes. In one aspect, the device housing comprises a substrate comprising one or more reservoirs or channels etched or embossed in the substrate. However, housing must be large enough and of sufficient width to be capable of forming the nanopore.

In certain aspect, material defining the opening of the nanopore may be coated to passivate the material or otherwise modify the electrical properties of the opening. For example, an organic or inorganic material may be deposited to reduce irregularities of the surface (e.g., resulting from the etching techniques used in fabrication of the nanopore). In one aspect, the coating comprises a biomolecule such as a protein. A nanochannel comprising the nanopore may be similarly coated. A nanochannel may be fully or partially coated.

In another aspect, the first and second reservoirs are separated by a membrane comprising the nanopore. One side of the membrane may be (or become) negatively charged, while the other side may be (or become) positively charged to drive a negatively charged molecule (such as a nucleic acid) through the nanopore.

The nanopore may be positioned anywhere on/through the housing. The nanopore may range in size from about 1 nm to as large as 300 nm, or from about 2 to about 20 nm, from about 1 to about 20 nm, from about 1 to about 10 nm, and from about 1-2 nm.
In one aspect, the aperture is large enough to restrict translocation of biopolymers to across the nanopore to a single molecule at a time. For example, the nanopore may be similar to the width of the biopolymer. In one aspect, the nanopore is large enough to permit single -stranded but not double-stranded nucleic acid molecules to pass through, while in another aspect, the nanopore is large enough to permit a single double-stranded or partially double-stranded molecule to pass through. For example, the nanopore comprises a diameter of about 2-20 nm, about 2-10 nm, or about 2-4 nm.

As shown in Figure 1, in one embodiment of the invention, the device comprises a channel 6 defining a channel wall in which a nanopore is positioned. In one aspect, this "nanochannel" 6 connects the first 3 and second reservoirs 4 of the device. Although the Figure shows a single contiguous housing defining the first and second reservoirs 3 and 4 and nanochannel 6, various types of housings or substrates to form these reservoirs and channel may be employed. For example, the first and second reservoirs may be part of a larger microfluidic or MEMs device comprising additional reservoirs and channels which may or may not communicate with the first and second reservoirs and nanopore channel. Additionally, first and second reservoirs may be connectable to one or more fluid delivery devices and other peripheral devices (e.g., pumps, tubing, capillaries, etc.). The device comprising the first and second reservoirs may comprise the same or different material as the housing or substrate defining the first and second reservoirs. The first and second reservoirs may be fabricated using any suitable process, such as micro- machining, casting, or thermoforming.

The housing may be machined from, or formed or cast into, a single piece of substrate material. Alternatively, the housing may be made of two more sections or plates. For example, a housing may comprise a top plate and a bottom plate, each comprising a portion of the first and second reservoir (e.g., a bottom half or top half), such that the first and second reservoirs are formed by mating the two plates. Alternatively, one plate may comprise the whole of the first and/or second reservoir and the other plate may for a cover for the reservoir(s). The plates may be removable from one another.

In certain aspects, the first and/or second reservoir communicate directly or indirectly with one or more connecting channels. The one or more connecting channels may be a variety of sizes and shapes. For example, the channel may be a microchannel having at least one dimension (e.g., such as width) less than 1 mm or a nanochannel having at least one dimension less than 1µm. Channels may be serpentine, straight, angled, or combinations of these configurations. In one aspect, as discussed above, the first and/or second reservoirs themselves may be configured as a channel. Connecting channels may supply reagents and/or buffers to the first and second reservoirs. Alternatively, or additionally, an inlet port communicating with the first and/or second channel may be used to supply reagents and/or buffers to the first and /or second reservoirs.

Conducting electrodes 7 may be provided on one or both sides of the nanopore to apply an electrical field across the nanopore. Electrodes 7 need not be located within the confines of the nanopore and do not need to be contiguous with the walls of the first and second reservoir, e.g., the electrodes may stand away from the walls.

A device electrode comprises an electrically conductive material. Electrically conductive materials include, but are not limited to, electrically conductive metals and alloys of platinum, iridium, palladium, gold, mercury, mercury calomel, tin, copper, zinc, iron, magnesium, cobalt, nickel, and vanadium and various combinations thereof. Electrochemical electrodes, such as Ag/AgCl or Hg/HgO electrodes may also be used. Other materials well known in the art that provide for electrical conduction may also be employed. Electrodes may vary in size and/or shape. To avoid formation of bubbles that may block or impede flow of biopolymer, electrodes may comprise materials, such as palladium, that aid in minimizing the formation of bubbles.

Movement of a biopolymer within the device may be due to electroosmotic flow of the liquid surrounding the biopolymer and/or to electrokinetic movement of the biopolymer within the liquid. This combination of electroosmotic movement and electrokinetic movement may include effects arising from the presence of spatial gradients in an electric field generated by electrodes 7.

The device 1 may also comprise one or more mechanisms for moving biopolymers. Such mechanisms may include, but are not limited to, mechanisms for providing electrophoretic flow, electroosmotic flow, electrokinetic flow, sheath flow, plug flow, parabolic flow, Hagen-Poiseuille flow, electrohydrodynamic flow, reptation, osmotic gradients, diffusion, electric fields, pressure and the like. Although device 1, is shown in Figure 1 with a pair of electrodes, additional electrodes may be provided for controlling movements of biopolymers in the first or second reservoir 3, and 4, and/or in one or more connecting channels or connecting reservoirs. In certain embodiments, first and second reservoirs communicate with capillaries or channels for performing electrophoretic separations such as capillary electrophoresis, on-chip separations. Such additional separation components may be used to simplify the complexity of a sample entering the first reservoir, for example. In certain embodiments, a reservoir may communicate with a medium for concentrating a sample (e.g., a solid phase with or without binding molecules, a filter, gel, etc.). In one aspect, the first and/or second reservoir communicates with a concentrating medium in a channel connected, directly or indirectly to the first and/or second reservoir.

Device electrodes are connectable to a voltage source 8, which may be positioned anywhere relative to the housing. Voltage sources 8 should be capable of establishing a voltage gradient between the respective electrodes to which they are connected. A variety of voltage sources may be employed with the present invention. In one aspect, the voltage source 8 comprises a polarity switchable voltage supply for reversing voltage after a desired number of biopolymers have translocated from first reservoir 3 to second reservoir 4. In certain embodiments, biopolymers may be translocated back from second reservoir 4 to first reservoir 4 by switching the polarity of a voltage source 8.

Additionally, an amplifier or detector may be used to detect changes in the ionic or electronic conductances across the aperture as a biopolymer traverses nanopore. In another aspect, the device is connectable to a detector 9 which can measure current over time. In a further aspect, a detector 9 measures electron flow across the nanopore using electrodes appropriately placed on either side of the nanopore.

Additional detectors may be provided. For example, a detector may be provided which can detect electrical or other properties of a fluid in one or more of the first or second reservoir or nanopore channel and/or may detect properties of molecules in the fluid (e.g., such as biopolymers). In one aspect, the detector may detect a label on a biopolymer (labeled directly or indirectly) in a solution in the first or second reservoir and/or nanopore channel. For example, the label may be a fluorescent label and the detector may be an optical detector. In one aspect, the label selectively labels biopolymers of a certain sequence. Such detectors may be used to detect the presence, amount and/or type of biopolymers in the reservoirs, and/or in connecting channels or other reservoirs in communication with the first and second reservoirs.

In a further aspect, one or both reservoirs and/or connecting channels comprise sensor(s) for sensing a condition with the reservoir(s) and/or connecting channels, e.g., such as temperature, pH, etc.

In still another aspect, the first and/or second reservoirs comprise heating element(s). Such elements may be used, where desired, to convert single-stranded nucleic acid molecules to double-stranded forms and/or to perform temperature-sensitive processes (e.g., such as amplification mediated by a thermostable polymerase).

In one embodiment, the invention provides a system comprising one of the devices described above in communication with a processor 10 for obtaining data from a detector 9 which monitors translocation of the biopolymer (e.g., by measuring current over time). In one aspect, the processor provides feedback to one or more device components, e.g., to change properties of an electric field, turn off a voltage supply, reverse polarity of voltage, implement functions of one or more sensors, detectors, heating elements, fluid or sample delivery devices, fluid or biopolymer collection devices and the like. Functions of the processor may be implemented at least in part by, in hardware, software, and/or combinations thereof.

In one aspect, the processor 10 has access to a memory. The memory may be a magnetic, optical, or solid state storage device (including magnetic or optical disks or tape or RAM, or any other suitable device, either fixed or portable). The processor may include a general purpose digital microprocessor suitably programmed from a computer readable medium carrying necessary program code, to execute all of the functions required of it as described above. Suitable programming can be provided remotely to the processor, or previously saved in a computer program product such as memory or some other portable or fixed computer readable storage medium. For example, a magnetic or optical disk may carry the programming, and can be read by disk reader in communication with the processor.

In one aspect, data collected by the processor (e.g., such a data relating to changes in current over time) can be stored in the memory. In another aspect, data relating time during which a voltage is to be applied to size of one or more biopolymers is stored in the memory (e.g., in a relational database). In a further aspect, data relating an amount of time after current impedance is observed to size of one or more biopolymers is stored in the memory. In still a further aspect, in response to notice of the passage of a predetermined time after current impedance, the processor 10 provides instructions to the voltage source 8 to stop applying voltage and/or to reverse polarity. Such notice may be provided when the detector 9 (which monitors current over time) sends a signal to the processor 10. In one aspect, the predetermined time is selected by a user. For example, the user may input the desired size of a molecule to be separated and/or isolated into an interface of a user device in communication with the processor and the time period associated with the size of the molecule (e.g., in the relational database) is identified by the processor. The detector 9 then notifies the processor 10 when the predetermined time has passed and the processor 10 then sends instructions to the voltage source 8, to stop applying the voltage and/or to reverse polarity of the voltage. In one aspect the detector 9 communicates with a timer/counter circuit that provides a signal to the processor 10. The timer/counter may be a circuit that is separate from the processor 10 or alternatively, may be part of the processor. In certain aspects, a ciock signal is sent to the processor 10 via a digital signal processor. In another aspect, the predetermined time is stored in the memory. In a further aspect, the predetermined time may be selected by the processor in response to feedback from one or more system components.

Other configurations of reservoirs and nanopores are possible and are encompassed within the scope of the invention.

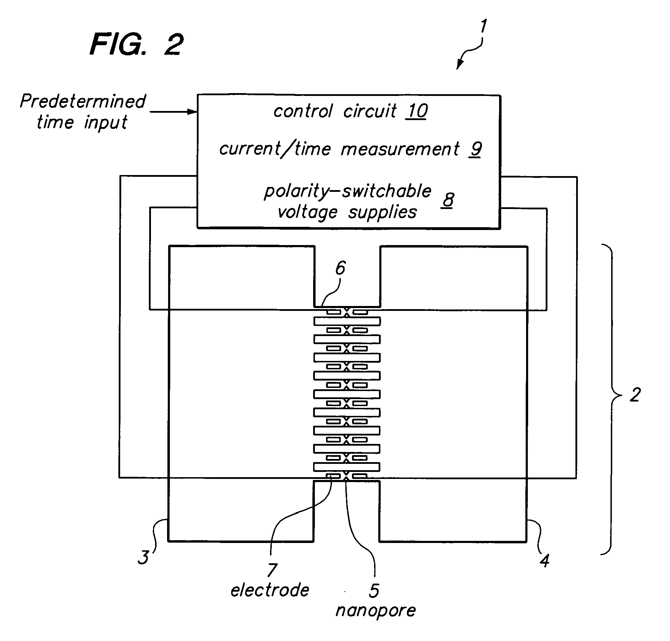

In one embodiment, as shown in Figure 2, the invention provides a device for performing parallel separations of biopolymers, thereby increasing throughput of the process. In one aspect, the device comprises a first reservoir 3 connected to a second reservoir 4 by a plurality of nanochannels 6, each comprising a nanopore 5. The device further comprises a plurality of electrode pairs 7, one for each nanopore 5. A processor 10 may be used to apply a voltage bias from voltage source 8 to the nanopores 5. In one aspect, such a device may comprise at least about 2, at least about 5, at least about 10, at least about 20, at least about 50, at least about 100 nanochannels 6. In another aspect, the device comprises a similar or different number of connecting channels that feed into the first and/or second reservoirs. A detector 9 monitors changes in current over time. Generally, the amplitude of these changes will be the same at each of the nanopores 5.

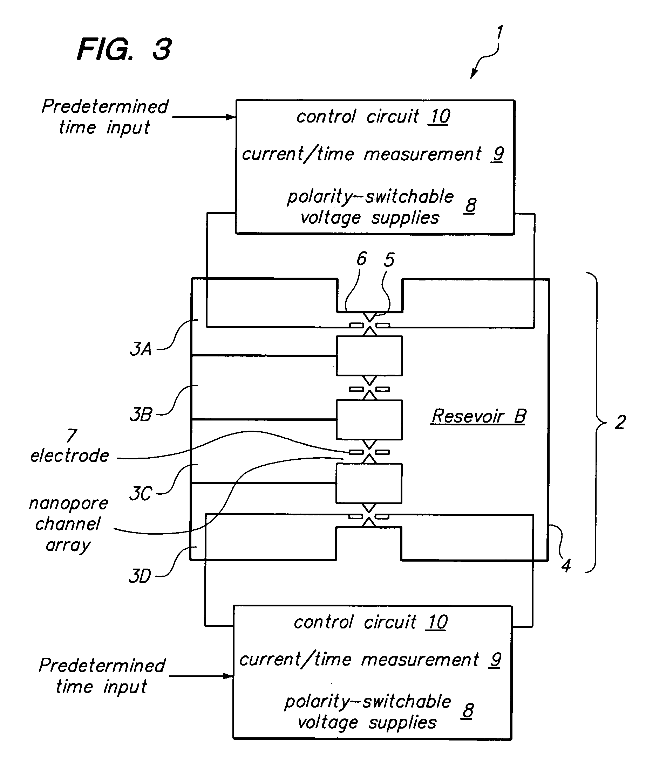

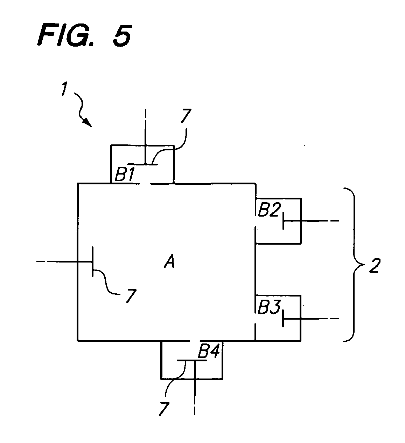

In another embodiment, as shown in Figure 3, the device may be configured for sorted separation. In one aspect, the device comprises a plurality of first reservoirs 3A, 3B, 3C, and 3D, each of which connect to a single second reservoir 4 via separate nanopore 5-comprising nanochannels 6. Each nanopore 5 comprises an associated electrode pair 7 (or each nanochannel 6 may form an electrode-not shown). At least two of the first reservoirs are independently controlled by a voltage supply 8 so that a voltage bias may be applied to one of the nanopores 5 independently of the voltage that is applied (or not applied) to the other nanopore 5. In one aspect, a voltage bias across each nanopore 5 is independently controlled. This embodiment permits biopolymers to be separated according to a plurality of characteristics, which may include different characteristics, while taking advantage of the high throughput permitted by providing multiple nanopores. Although Figure 3 shows that sorting may occur into a single reservoir (reservoir B), as shown in Figure 5, in certain aspects, device is provided which may sort biopolymers into two or more reservoirs according to a plurality of characteristics. For example, a mixed population of biopolymers (comprising at least two different characteristics such as different sizes) may be provided in a first reservoir A and sorted into subreservoirs B1, B2, B3, B4, etc, each of the subreservoirs in fluid communication with the first reservoir by a nanopore 5. In one aspect, the voltage biases across the two or more reservoirs are independently controlled and molecules sorted into the two or more subreservoirs may comprise different characteristics.

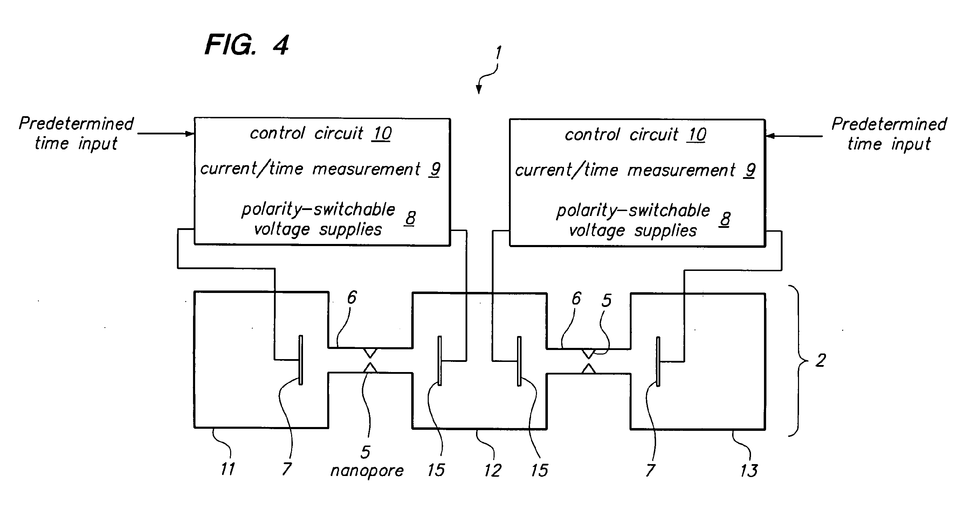

In a further embodiment, as shown in Figure 4, the device is configured for tandem separation. In one aspect, the device comprises at least three reservoirs, at least one of which is connected to two others by two nanopores. For example, Figure 4 shows three reservoirs 11, 12, and 13 arranged and connected in series by nanochannels 6. Each nanopore 5 in a nanochannel 6 may be subjected to an electric field generated by a pair of electrodes, one on either side of the nanopore 5. Thus, reservoirs serving as "bridging reservoirs" connecting two other reservoirs may comprise two electrodes 7, one on each side of a nanopore 5. A voltage bias may be applied to each nanochannel 6 and voltages at each nanochannel 6 may be independently controlled. For example, a voltage bias applied for a predetermined time across electrode pair 14 may be used to control polymer translocation between a reservoir 11 and 12 to separate biopolymers having a first size and a voltage bias applied for a predetermined time (e.g., a different predetermined time) across electrode pair 15 may be used to separate polymers having a second size (e.g., a different size from the first size) and translocate these from reservoir 12 to 13. The device may be modified to include more reservoirs to separate multiple compounds. In one aspect, the at least three reservoirs 11, 12, and 13 comprises one or more inlet and/or outlet ports to remove sample/fluid/reagents from reservoir(s) and/or to add sample/fluid/reagents to reservoir(s). One or more reservoirs may comprise one or more connecting channels, as described above. In some aspects, one or more reservoirs may perform one or more system functions in addition to biopolymer translocation. For example, amplification reactions, enzyme digestions, hybridization or other binding reactions, sample concentration, etc. may be performed within one or more of the reservoirs. Additionally, or alternatively, the one or more system functions may be performed in one or more connecting channels or reservoirs which do not comprise nanopores but which are connectable to reservoirs that do comprise nanopores.

In still further embodiments, devices may be configured to perform a combination of parallel, sorted and/or tandem separations. Sorted separations may be from a plurality of reservoirs into a single reservoir or from a single reservoir into a plurality of different reservoirs or some combination thereof.

### Methods of Using Nanopore-Based Separation Devices

In one embodiment, the invention provides methods for using nanopore-based separation devices such as those described above. In one aspect, the method comprises providing a plurality of biopolymers (e.g., biopolymers comprising different numbers of monomers) in a first reservoir 3 connected to a second reservoir 4 by a nanochannel 6 comprising a nanopore 5, establishing an electrical field across the channel 6sufficient to translocate a biopolymer across the nanopore 5, thereby separating the biopolymer from other biopolymers, and collecting the translocated biopolymer in the second reservoir 4 or collecting the remaining biopolymers in the first reservoir 3.

The method may be used to separate the same type of biopolymers or different types of biopolymers. In one aspect, the biopolymer comprises a nucleic acid molecule. In another aspect, the biopolymer comprises a polypeptide molecule. In a further aspect, the method is used to separate both nucleic acid and polypeptide molecules, e.g., the method may be used to separate nucleic acid molecules from polypeptides or nucleic acids and polypeptides having certain characteristics (e.g., size) from nucleic acids and polypeptides having other characteristics.

Generally, the first and second reservoirs 3 and 4 comprise a medium, such as a fluid, that provides biopolymers with adequate mobility to translocate from one reservoir to another across the nanopore 5. In one aspect, the medium comprises an electrically conductive medium, such as an electrolyte solution. For example, the medium may comprise ions (e.g., sodium, potassium, chloride, calcium, cesium, barium, sulfate, or phosphate) capable of carrying an electric current.

A voltage difference can be imposed across the nanopore 5 between the first and second reservoirs 3 and 4. The size of the voltage bias may be selected to concentrate biopolymers of a certain size at the nanopore 5. Exemplary voltages may range from about 20 millivolts to a Volt; however, voltages may be optimized to suit particular applications.

Conductance across the nanopore 5 can be determined by measuring the flow of current across the nanopore 5 via the conducting medium. As a biopolymer translocates through a nanopore 5, ionic current is impeded and a decrease of measured ion current is observed. For a given system and applied voltage, the translocation velocity (number of monomers in a polymer translocating per second, e.g., number of nucleotide bases or amino acids per second) is approximately constant and therefore translocation time is proportional to the length of the polymer. Thus, the duration of impeded ion current is proportional to the length and size of the biopolymer. After a biopolymer passes through a nanopore 5, ion current returns to an unimpeded level, e.g., the level observed prior to translocation of the biopolymer. In one embodiment of the invention, an electric field is applied across the nanopore 5 for a sufficient amount of time to translocate a biopolymer of a selected size across the nanopore 5, to separate that biopolymer from other biopolymers in a sample population of biopolymers. Current can be measured in real time, e.g., by a detector 9 and data relating to current changes over time and voltage provided to a processor 10.

In one aspect, in response to data relating to current changes, the processor 10 provides instructions to the system to alter voltage at a voltage source 8. For example, the processor 10 can provide instructions to the voltage source 8 to reverse polarity. In one aspect, the processor 10 receives notice of the passage of a predetermined time, reflecting the translocation time needed to separate a biopolymer comprising characteristics of interest (e.g., a desired number of monomers), in response to which notice, the processor 10 provides instructions to the voltage source 8 to alter properties of the voltage bias applied across the nanopore 5.

The processor may receive notice of the passage of time from a timer mechanism such as a counter/timer circuit in communication with the detector 9 and/or may have, or be in communication with processing components (e.g., such as a microprocessor comprising a counter/timer) that are able to monitor and provide information to the processor 10 relating to time intervals. In one aspect, if the passage of time corresponds to the predetermined time may then provide instructions to the voltage source 8 as discussed above. The length of time constituting the predetermined time may be set by a user, e.g., by inputting the time period into a display of a user device in communication with the processor 10 (either directly, or through a network or server), and/or by instructions from a computer readable medium (e.g., a computer program product) which includes the predetermined time and are executed by the processor. Alternatively, or additionally, the predetermined time may be set in response to feedback from one or more system components (e.g., such as a sensor in communication with one or more reservoirs of the device).

In one aspect, once a biopolymer begins to translocate across a nanochannel 6 (e.g., once a decreasing ion current is detected), a detector 9 begins to record time of the impeded ion current ("translocation time"). If the translocation time is less than a predetermined time, the polarity of the applied voltage remains the same and the polymer passes through the nanopore 5 from the first reservoir 3 to the second reservoir 4. The process then repeats, supplying another biopolymer to the second reservoir 4. When the recorded translocation time exceeds the predetermined time, the processor 10 provides instructions to the voltage source 8 to switch polarity of the voltage supply. When this happens, the polymer will reverse its direction of translocation and travel back to the first reservoir 3. Once the unimpeded current level is achieved (i.e., the biopolymer is back in the first reservoir 3, the processor 10 provides instructions to the voltage source 8 to switch back to the initial polarity. The process may be repeated any number of times. After a certain separation time, while the first reservoir may still comprise biopolymers of different monomer size, substantially all of the biopolymers in the second reservoir 4 comprise biopolymers whose translocation time is less than the predetermined time.

In one aspect, the predetermined time is set to achieve the following separation: only biopolymers with the smallest number of monomers translocate to the second reservoir 4, while only biopolymers with the largest number of monomers remain in the first reservoir 5

A plurality of biopolymers may be separated by providing a plurality of parallel nanopores (e.g., as shown in Figure 2). Additionally, biopolymers may be separated according to a plurality of different characteristics (e.g., such as different lengths) at a time, for example as shown in Figure 3. Sequential sorting of biopolymers may be performed by providing a plurality of reservoirs in tandem as shown in Figure 4, for example. Combinations and variations of these approaches may be used and are encompassed within the scope of the invention.

In one aspect, the method comprises the step of removing, concentrating and/or isolating biopolymers from one or more reservoirs after separation has occurred.

By requiring the dip in current to occur after a certain period in time after appl ication of a voltage, biopolymers of a desired size may be selected for translocation through the nanopore. The longer the biopolymer, the longer it will take to translocate through the nanopore. The time required to translocate a biopolymer of a selected size may be determined empirically using standard biopolymer molecules of known size. Thereafter, a voltage may be applied to biopolymers of unknown size to separate these according to size in the first and second reservoirs.

Methods of the invention may be used in a variety of applications that exploit the separation of biomolecules.

In one aspect, the methods enable discrimination of nucleic acid molecules comprising insertions, deletions, or combinations thereof. For example, the method may be used to separate nucleic acids that differ by 1 base pair in length or more. In one aspect, the method may be used to separate nucleic acids that differ in size by 1% of the total number of base pairs in the nucleic acid molecule. The size of nucleic acid molecules to be separated may vary and in one aspect, may range from 1 to 100,000 base pairs or greater.

Accordingly, methods, devices and systems of the invention are useful assays for separating allelic variants (e.g., such as mutations) which differ in size. The methods may also be used to separate and isolate amplification products (e.g., such as PCR products) or other primer extended products, in RFLP analysis, in separating and isolating cloning products (e.g., such as ligated nucleic acids), in separating polypeptides, in separating double-stranded or partially double-stranded molecules from single stranded molecules, in separating molecules bound to other molecules (e.g., nucleic acids bound to other nucleic acids, nucleic acids bound to polypeptides, polypeptides bound to other polypeptides, and the like), in separating processed forms of molecules from non-processed forms (e.g., cleaved proteins from non-cleaved proteins, digested nucleic acid molecules from non-digested molecules, spliced RNA molecules from non-spliced molecules), in separating modified from unmodified biopolymers, in separating chromosomes according to size, in separating rearranged genes from non-rearranged genes (e.g., by separating fragments comprising rearranged genes from fragments comprising nonrearranged genes), in separating and isolating plasmid DNA molecules from genomic DNA molecules, and the like.

Separated molecules may be further processed, e.g., by concentration, characterization (e.g., sequencing), isolation, and the like. In certain embodiments, separation of bound molecules from unbound molecules may be used to identify receptors, ligands, or sequences that interact with each other. For example, a first population of biopolymers may be contacted with a second population of biopolymers (which may be labeled with a detectable label and/or affinity tag) and binding complexes separated from biopolymers that are unbound using a nanopore device according to the invention. Binding partners may be separated from each other (e.g., separating labeled or tagged molecules from unlabeled or untagged molecules) using the nanopore device or by another method and the labeled/tagged molecules or the unlabeled/untagged molecules evaluated further by sequencing or another suitable assay (e.g., such as mass spectrometry). Binding partners may be used as probes in certain embodiments, e.g., known nucleic acid sequences may be used to select and separate complementary nucleic acids from non-complementary nucleic acids. Similarly, polypeptides (e.g., antibodies or antigen-binding fragments) may be used as probes for other polypeptides (e.g., such as antigens). In certain embodiments, nucleic acids are used as probes for polypeptides and visa versa (e.g., nucleic acid molecules may be used to probe a sample for transcription factors or other nucleic acid binding molecules).

Linker molecules and/or tags may be selectively associated with biopolymers (e.g., binding or associating with biopolymers of a selected sequence), thereby increasing the size of the biopolymer, and used to separate biopolymers of the selected sequence from those that do not comprise the selected sequence.

In certain aspects, the processor 10 obtains data relating to characteristics of biopolymers in one or more reservoirs (e.g., such as the sequence of a biopolymer, its ability or inability to bind to other molecules, its size, and the like). Such data may be obtained from or added to a memory in communication with the processor. In certain embodiments, a user of the system inputs data relating to characteristics of biopolymers in one or more reservoirs into the display of a user device in communication with the processor. This may be done remotely or locally (e.g., in the same room where the nanopore device is located). In additional or alternative embodiments, the user may view data relating to biopolymers in one or more reservoirs on the display of the user device.

Separated molecules also may be quantified. For example, an amount of biopolymers that have translocated into a reservoir and/or which remain in a first reservoir after translocation of biopolymers into a second reservoir may be determined and compared to a number of biopolymers that were in the non-separated sample.

This may provide a measure of expression of natural, mutated and/or pathogenic nucleic acids; spliced, unspliced or variently spliced RNA biopolymers; processed and/or unprocessed biopolymers; modified or unmodified biopolymers.

Other assays which may exploit separation steps may be performed using devices, methods and systems according to the invention and are encompassed within the scope of the invention.

### Kits

Kits for use in biopolymer separation assays are provided. The subject kits at least include a nanopore device of the subject invention. The kits may further include one or more additional components necessary for carrying out the assay, such as sample preparation reagents, buffers, labels, enzymes, control biopolymers (e.g., of known sizes) and the like. As such, the kits may include one or more containers such as vials or bottles, with each container containing a separate component for the assay. The kits typically further include instructions for how practice the subject separation assays according to the subject invention, where these instructions are generally present on at least one of a package insert and the package of the kit. In one aspect, the kit comprises one or more computer program products according to the invention.

All references cited herein are incorporated herein in their entirety by reference and for all purposes to the same extent as if each individual publication or patent or patent application was specifically and individually indicated to be incorporated by reference in its entirety for all purposes.

The disclosures in United States patent application No. 10/959,442, from which this application claims priority, and in the abstract accompanying this application are incorporated herein by reference.

## Claims

1. A method for separating biopolymers, comprising:
providing a plurality of biopolymers in a first reservoir (3) connected to a second reservoir (4) by a nanopore (5);
establishing an electrical field across the nanopore sufficient to translocate a biopolymer across the nanopore, thereby separating the biopolymer from other biopolymers; and
collecting the translocated biopolymer in the second reservoir or biopolymers in the first reservoir.

2. A method as claimed in claim 1, wherein the biopolymer is bound to another molecule and the method comprises translocating bound biopolymer and/or wherein the biopolymer is bound to another molecule and the method comprises translocating unbound biopolymer.

3. A method as claimed in claim 1 or 2, wherein the first reservoir (3) and the second reservoir (4) are connected by a plurality of nanopores (5).

4. A method as claimed in claim 1, 2 or 3, wherein the first reservoir (3) comprises a plurality of subreservoirs, each subreservoir connected to the second reservoir (4) by a nanopore (5).

5. A method as claimed in claim 4, wherein an electric field is applied independently to at least two of the nanopores (5).

6. A method as claimed in claim 4 or 5, wherein biopolymers having different characteristics are translocated through at least two of the nanopores (5).

7. A method as claimed in claim 6, wherein the different characteristics comprise different sizes.

8. A method as claimed in any preceding claim, wherein an electric field is applied for a sufficient amount of time to translocate a biopolymer of a selected size.

9. A method as claimed in claim 8, further comprising collecting a plurality of biopolymers of the selected size in the second reservoir (4).

10. A method as claimed in claim 8 or 9, wherein the biopolymer is a nucleic acid and the biopolymer of the selected size is less than about 100 base pairs different, less than about 20 base pairs different or less than about 10 base pairs different in size from biopolymers remaining in the first reservoir (3).

11. A method as claimed in any preceding claim, further comprising monitoring current across the nanopore (5) over time.

12. A method as claimed in any preceding claim, wherein the electric field is established by applying a voltage bias across the nanopore (5) and the method further comprises reversing polarity of the applied voltage after a period of time sufficient to translocate a biopolymer of a selected size into the second reservoir (4).

13. A method as claimed in claim 12, wherein the voltage bias is controlled by a processor (10) which receives input relating to passage of time after a current across the nanopore (5) begins to decrease below a threshold amount, and provides instructions to a voltage source (8) applying the voltage after a predetermined time sufficient to translocate the biopolymer of the selected size, to stop application of the voltage or to reverse polarity of the applied voltage.

14. A method as claimed in claim 13, wherein the step of applying the voltage for the predetermined time is repeated one or more times.

15. A method as claimed in any preceding claim, wherein a plurality of biopolymers is translocated into the second reservoir (4) through one or more nanopores (5) connecting the first reservoir (3) and the second reservoir and wherein the method further comprises the step of concentrating translocated biopolymers.

16. A method as claimed in any preceding claim, wherein the method is used to separate allelic variants, to separate amplification products, to separate nucleic acid fragments generated by contacting nucleic acid samples with a nuclease, to separate ligated nucleic acid molecules from non-ligated nucleic acid molecules, to separate and/or isolate cloning intermediates, to separate processed biopolymers from unprocessed biopolymers, to separate modified biopolymers from unmodified biopolymers, to separate spliced RNA molecules or cDNA copies thereof from unspliced or alternatively spliced molecules, to separate chromosomes of different size, to separate plasmid DNA from genomic DNA, and/or to separate rearranged genes from non-rearranged genes.

17. A method as claimed in any preceding claim, wherein the second reservoir (4) is connected to a third reservoir by a nanopore (5) and the method comprises establishing an electrical field across the nanopore connecting the second reservoir and the third reservoir sufficient to translocate a biopolymer from the second reservoir across the nanopore into the third reservoir.

18. A device comprising
a first reservoir (3) connectable to a second reservoir (4) by a nanopore (5);
a voltage source (8) for generating an electric field across the nanopore; and a
a processor (10) in communication with the voltage source; wherein the processor provides instructions to the voltage source to regulate the time period during which voltage is applied.

19. A device as claimed in claim 18, wherein the first reservoir (3) is connectable to the second reservoir (4) by a channel (6) comprising the nanopore (5).

20. A device as claimed in claim 18 or 19, wherein the first reservoir (3) is connectable to the second reservoir (4) by a plurality of nanopores (5).

21. A device as claimed in claim 20 wherein voltage across each nanopore (5) is independently controlled.

22. A device as claimed in any of claims 18 to 21, wherein either or both of the first reservoir (3) and the second reservoir (4) comprise an outlet port for removing biopolymer and/or wherein the first reservoir and/or second reservoir comprise channels.

23. A device as claimed in any of claims 18 to 22, wherein the device includes a connecting channel in fluid communication with the second reservoir (4).

24. A device as claimed in any of claims 18 to 23, wherein the first reservoir (3) comprises a plurality of subreservoirs, each subreservoir connectable to the second reservoir (4) by a nanopore (5) and/or wherein the second reservoir comprises a plurality of subreservoirs, each subreservoir connectable to the first reservoir by a nanopore.

25. A device as claimed in claim 24, wherein a voltage may be independently applied across nanopores (5) of at least two of the subreservoirs.

26. A device as claimed in claim 24 or 25, wherein each of the subreservoirs comprises an outlet port for removing a sample.

27. A device as claimed in any of claims 18 to 26, wherein the device further comprises a third reservoir connectable to the second reservoir (4) by a nanopore (5) and a voltage may be applied independently across nanopores (5) connecting the first reservoir (3) and the second reservoir and the second reservoir and the third reservoir.

28. A device as claimed in any of claims 18 to 27, wherein the device further comprises an electrode (7) on either side of the nanopore (5).

29. A computer program product comprising a computer readable medium carrying program code for executing functions by the processor of the device of any of claims 18 to 28.

30. A kit comprising the device of any of claims 18 to 28 and a biopolymer.

31. A kit as claimed in claim 30, wherein the kit comprises a biopolymer of known size or a plurality of biopolymers of known sizes.

32. A system comprising a device as claimed in any of claims 18 to 31, and a memory comprising a relational database that comprises data relating current impedance to size of biopolymers, and wherein the processor has access to the memory.
